# EUROPEAN PATENT SPECIFICATION

(11) **EP 3 796 908 B1**
(45) Date of publication and mention of the grant of the patent: **10.05.2023**
(21) Application number: 18940434.6
(22) Date of filing: 16.11.2018
(51) Int. Cl.: A61K 31/445, A61K 9/22, A61P 13/00, A61K 9/20, A61K 9/28

(54) **CONTROLLED RELEASE PROPIVERINE FORMULATIONS**
PROPIVERIN-FORMULIERUNGEN MIT KONTROLLIERTER FREISETZUNG
FORMULATIONS DE PROPIVÉRINE À LIBÉRATION CONTRÔLÉE

(43) Date of publication of application: 31.03.2021
(73) Proprietor: Santa Farma Ilaç Sanayi A.S., 34382 Istanbul (TR)
(72) Inventor: YILDIRIM, Ersin, 41455 Dilovasi /Kocaeli (TR); KANIK, Bayram, 41455 Dilovasi /Kocaeli (TR); AKTAS, Tansel, 41455 Dilovasi /Kocaeli (TR); ÖZTÜRK, Fatma, 41455 Dilovasi /Kocaeli (TR); ÖZYÜREK, Filiz, 41455 Dilovasi /Kocaeli (TR)
(74) Representative: Bulut, Pinar
(86) International application number: PCT/TR2018/050698
(87) International publication number: WO 2020/101586

(56) References cited:
- WO-A1-2017/026950
- CN-A- 103 517 708
- US-A1- 2009 258 066
- Anonymous: "EUDRAGIT@ Acrylic Polymers for Solid Oral Dosage Forms", Evonic Industries, 1 February 2017 (2017-02-01), pages 1-16, XP055707168,

## Description

### Field of the Invention

The invention relates to controlled release pharmaceutical compositions for oral administration comprising propiverine or a pharmaceutically acceptable salt thereof, wherein pharmaceutical composition has an enteric coating in specified amount of range by weight of core tablet.

### Background of the Invention

Propiverine (1-methyl-4-piperidyl diphenylpropoxy acetate), a benzylic acid derivative as shown in Formula I, 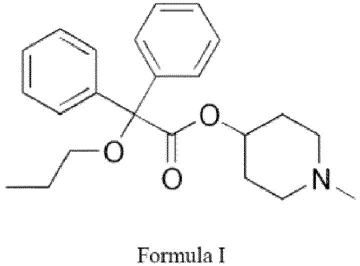 is an antimuscarinic agent.

An official monograph for propiverine hydrochloride is available in Japanese Pharmacopoeia XVI (2011).

Propiverine is a BCS (Biopharmaceutical Classification System) Class III drug, which exhibits low permeability and high solubility.

Propiverine is indicated in adults for the symptomatic treatment of urinary incontinence and/or increased urinary frequency and urgency in patients with overactive bladder syndrome.

A therapeutic agent for an overactive bladder syndrome, a medical ingredient is preferably released in the intestinal tract. Overactive bladder is an essential syndrome that presents urinary urgency. The first line drugs which have the anticholinergic activity, are treated overactive symptoms. Propiverine is widely used for demonstrating to both anticholinergic and calcium-modulating properties.

Propiverinein the form of hydrochloride salt, iscurrently on the market as immediate release and modified (extended) release by ApogephaArzneimittel GmbH. The immediate release (IR) tablets contain 15mg propiverine hydrochloride (equivalent to 13.64 mg propiverine per tablet), whereas the modified release capsules contain 30 mg and 45 mg propiverine hydrochloride (equivalent to 27.28 mg propiverine per capsule, equivalent to 40.92 mg propiverine per capsule).

From the gastrointestinal tract, propiverine is nearly completely absorbed and undergoes extensive first pass metabolism. Effects on urinary bladder smooth muscle cells are due to the parent compound and three active metabolites as well, which are rapidly excreted into the urine. After oral administration for immediate release of propiverine is absorbed from the gastrointestinal tract with maximal plasma concentrations reached after 2.3 hours and the mean absolute bioavailability is 40.5%. Whereas for modified release of propiverine is absorbed from the gastrointestinal tract with maximal plasma concentrations reached after 9.9 hours and the mean absolute bioavailability is 60.8 ± 17.3%.

Food does not influence the pharmacokinetics of propiverine. The bioavailability of propiverine after the meal was 99% compared to the fasting conditions. Administration of the ER capsule leads to mean Cmax - concentrations of propiverine of about 70 ng/ml reached within 9.5 hours after administration. The Cmax values for the main metabolite propiverine -N-oxide were slightly increased by food (f= 1.26), whereas the extent of absorption was unchanged. Propiverine-N-oxide showed for all pharmacokinetic parameters 90 % confidence intervals within the acceptance ranges. An adjustment of dose in relation to food intake is not required. DD 106643 numbered patent is the basic patent that is first mentioned about propiverine and its salts.

EP 1435915 (ApogephaArzneimittelGmbh) provides an prolonged-release pharmaceutical composition comprising 4mg-60mg of propiverine and/or one or several acceptable salts thereof, where the composition is adapted for once-a-day oral administration and has specific in vitro release characteristics measured in 750 ml of 0.1 N hydrochloric acid during the first hour and subsequently measured in 750 ml USP buffer at pH=5.8 using a Ph. Eur. basket method at 100 rpm and 37°C.

WO 2006/046560 (Taiho Pharmaceutical) provides oral preparation that ensures good sensation upon administration thereof obtaining a suspension resulting from mixing of propiverine hydrochloride, a surfactant, a pH adjusting agent and water and having its pH value adjusted between 6.5 and 8.0, and subsequently granulating the suspension together with common additives for preparation.

EP 2276472 (Eurand) provides a phamaceutical compositions by comprising a plurality of controlled-release particles, wherein a core comprising a weekly basic drug (e.g. propiverineas given in Examples 5-7), an alkalline buffer layer disposed over the core and a water insoluble polymer of the controlled release coating disposed over the alkalline buffer layer. Enteric effect is provided by multiple coatings.

CN 102579404 A (Guangzhou Coredes) discloses a sustained-release capsule containing propiverine hydrochloride. The sustained capsule comprises micropills which have uniform particle size and micropills comprise pill cores containing drug accounting for 75 to 97 percent and the sustained release layers accounting for 3 to 25 percent by weight percentage.

WO 2012/154892 (Theravida) disclosesa pharmaceutical compositions comprising a therapeutically effective amount of extended release propiverine, or a pharmaceutically acceptable salt thereof, and a therapeutically effective amount of pilocarpine, or a pharmaceutically acceptable salt thereof.

WO 2007/105229 (Panacea BiotecLtd) provides a novel controlled release pharmaceutical composition comprising at least one active agent(s), at least one pH dependent polymer(s), at least one pH independent polymer(s) and at least one channel forming agent(s), optionally with other pharmaceutically acceptable excipients, wherein the ratio of the pH dependent polymer and the pH independent polymer is 1 : 10 to 10:1.

EP 173928 (Ab Leo) provides a controlled-release pharmaceutical preparation having biphasic release profile, comprising a drug tablet and a coating applied thereon, wherein the coating consists of a film-forming polymer which is insoluble in water and gastro-intestinal fluids and a water-soluble pore-creating material being randomly distributed in said polymer characterized in that the pore-creating material includes a drug active substance in a therapeutically effective amount.

US 5,695,781 and US 6,083,532 (Hallmark Pharmaceuticals, Inc., Duramed Pharmaceuticals, Inc.) disclose a three-component release rate controlling matrix composition that compose of a pH dependent and independent gelling polymer and an enteric polymer component.

EP 2851073 (Taiho Pharmaceutical CO LTD) provides a superior prophylactic agent and/or therapeutic agent comprising an effective amount of 4-piperidyl diphenylpropoxyacetate(propiverine) or a salt thereof, and a pharmaceutical carrier for treating stress urinary incontinence.

EP 3331502 A1 (Santa Farma San. A.S.) provides a controlled release pharmaceutical compositions for oral administration comprising propiverine or a pharmaceutically acceptable salt thereofhaving biphasic in vitro release characteristics wherein said composition also comprises at least one hydrophic polymer, at least one water soluble pore forming agent and at least two pH dependent polymer in a matrix formation. This composition provides a dissolution profile of propiverine less than 50.0% after nine hours and more than 85.0% after 24 hours as measured by USP Type I apparatus (basket) at 100 rpm, in 750 mL of 0.1 N hydrochloric acid for the first hour, subsequently in 750 mL USP buffer at pH 5.8 until nine hours, and subsequently in 750 mL of pH 6.8 USP buffer until 24 hours.

WO 2017/026950 A1 (SANTA FARMA) discloses tablets, the core comprising propiverine, lactose, HPC, HPMC, HPC, Eudragit L100 and Eudragit L100-55 for achieving a biphasic dissolution. Tablets may be coated in a conventional manner, such as with acyclic and/or methacrylic co polymers.

CN 103 517 708 A (BRKICIC CVJETKO; SOVIC BRKICIC LJILJANA) discloses Eudragit S100 as enteric coating in oral medication, with propiverine disclosed as active ingredient.

The present invention is related to a controlled release matrix form tablet design comprising propiverine or its acceptable salt thereof as active ingredient with a dedicated range of enteric coating by weight. The controlled-release effect is provided with the matrix composition and the coating.

### Summary of the Invention

Based on the prior art, the object of the invention is related with pharmaceutical formulations comprising an anti-muscarinic agent in a controlledrelease formulation.

It is an object of the present invention relates to pharmaceutical formulations comprising of propiverinein the matrix form presenting controlled release with pH dependent polymers and a specified range of the enteric coating by weight of the core tablet.

It is a further object of the present invention relates to an oral dosage form in the form of tablet comprising propiverine or one of its pharmaceutically acceptable salts wherein the release of activeingredient is controlled by tablet matrix and enteric coating in the intestine region.

In accordance with the present invention, the formulation provides the excellent binding and gelling with using coating agents such as Eudragit S100 in the coating formulation and Eudragit L100 and Eudragit L100-55 in matrix form of the core. After taken of the drug, dissolution is negligible at the low pH levels in the stomach. The enteric coating comprising Eudragit S100 is effective and stable enteric coatings with a fast dissolution in the upper bowel. The coating begins decomposing as Eudragit S100 is a pH dependent polymer which is soluble in upper bowel. As the composition continues to decompose this time the pH dependent polymers in the core which are in matrix form begin to be solubilized. Moreover, they are getting active in small intestine which has the pH range of 6 to 7.4.

It is surprisingly realized that the coating amount is effective in the dissolution of the pharmaceutical product in vivo studies. A specified range of pH dependent polymer comprising coating which is 0.5% to 2.5% is the effective range to get suitable in vivo results.

### Detailed Description of the Invention

Although propiverine, being a BCS Class III drug, shows high solubility independent of the pH of the aqueous media, the release properties of the formulation have been modified by using various polymers having different pH dependency.

The present invention provides an oral pharmaceutical composition comprising propiverine or a pharmaceutically acceptable salt thereof having biphasic in vitro release characteristics, which includes at least one hydrophilic polymer, at least one water-soluble pore-forming agent, at least twopH-dependent polymer in the core matrix composition which are effective at or above pH 5.5 and at or above pH 6.0 and a pH dependent polymer which is effective at or above pH 7.0 placed in the coating composition.

An enteric coating layer comprises at least one lubricant, at least one film coating agent and at least one pH - dependent polymer dissolving at or above pH 7.0.

For purposes of the present invention the term"controlled release" refers to a pharmaceutical dosage form which releases active ingredient(s) over a prolonged period of time, in this case over a period up to 24 hours, which also be referred to as"prolonged release","extended release", "modified release", "delayed release" and "sustained release". When used in association with the dissolution profiles discussed herein, the term"controlled release" refers to that portion of a dosage form made according to the present invention which delivers active ingredient(s) over a period of time, at least up to 24 hours. Preferably the present invention is a controlled release oral pharmaceutical formulation and provides a release of the drug at least up to 24 hours.

Intended dissolution rates in the specific pH conditions were achieved with combination of at least three pH dependent polymers, at least one pore forming agent and at least one hydrophilic polymer. During dissolution, the water enters to the tablet through soluble pore forming excipients as lactose monohydrate and water soluble polymers tend to thicken to form gel as they absorb the water. The forming gel enables the tablet to release propiverine in a slower release. In the said invention water soluble pore forming excipients play a significant role for gel forming as well as gel forming pH independent polymers.

At the present invention, enteric coating shows an effect of the biocompatible of the drug with the amount of the coating layer that changes from 0.5% to 2.5%by weight of the core tablet.

In the preferred embodiment of the invention, the pharmaceutically acceptable polymer is a hydrophilic polymer. Preferably, the hydrophilic polymer is selected from polyvinyl pyrrolidine, hydroxypropyl cellulose, hydroxypropyl methyl cellulose, methyl cellulose, vinyl acetate copolymers, polyethylene oxide, carbomer, polysaccharides (such as alignate, xanthan gum, carrageenan etc.) polyethylene oxide, maleic anhydride/methyl vinyl ethercopolymers and derivatives and mixtures thereof. More preferably, the hydrophilic polymer is selected from hydroxypropyl cellulose, hydroxypropylmethyl cellulose and derivatives and combination thereof.

In the preferred embodiment of the present invention, the pharmaceutically acceptable pore forming agent is water soluble. Any water soluble material present in the coating which dissolves and forms pores in the coating layer may act as pore forming agent. Preferably, the water soluble pore forming agent is selected from potassium chloride, sodium salts, calcium salts, magnesium salts, amino acids, weak acids, carbohydrates (such as sucrose, mannitol, sorbitol), lactose (hydrous, anhydrous, monohydrate, spray dried), polymers with amino and/or acid, microcrystalline cellulose, silicified microcrystaline cellulose, starch, pregelatinised starch, modified starch, calcium phosphate dihydrate, dibasic calcium phosphate anhydrous, tribasic calcium phosphate, calcium sulphate trihydrate, calcium sulphate dihydrate, calcium carbonate, kaolin, cellulose acetate, powder cellulose, xylitol, polydextrose or polyvinyl pyrrolidine. More preferably, the water soluble pore forming agent is lactose (hydrous, anhydrous, monohydrate, spray dried).

In the controlled release propiverine formulations of the present invention, it is found out that besides matrix form the solubility features are dependent on the amountof enteric coating on tablet.

The present invention comprises pH dependent polymers that are soluble at various, or different in the case of more than one pH. The said formulation comprises pH dependent polymers to achieve a reduced release in lower pH values. The pH dependent polymers stay stable in the low pH values while in the higher pH values, start to dissolve and release propiverine to the dissolution medium. The pH dependency of the polymers and their ratios has significant effect on the attainment of required release characteristics in the modified release pharmaceutical formulations.

In one embodiment of the invention, the pharmaceutical composition for the tablet comprises at least two pH dependent polymers comprising a mixture of at least one pH dependent polymer dissolving at or above pH 5.5 and at least one pH dependent polymer dissolving at or above pH 6.0 and besides the pharmaceutical composition for the enteric coating comprises at least one pH dependent polymer dissolving at or above pH 7.0.

The pH dependent polymer may be selected from the group of cellulose acetate, cellulose acetate phthalate, cellulose acetate trimellitate, cellulose acetate succinate, hydroxypropylmethylcellulose (hypromellose) phthalate, hydroxypropyl phthalate, hydroxypropyl methyl cellulose acetate phthalate, hydroxypropyl methylcellulose (hypromellose) acetate succinate, polyvinyl acetate phthalate, shellac, co -polymerized methacrylic acid/methacrylic acid methyl esters such as, materials known under the trade name EUDRAGIT^{®} L12.5, L100, L30D-55, L100-55, S100, S12.5 and derivatives and mixtures thereof.

EUDRAGIT^{®} L100 is described at the Ph.Eur (Methacrylic Acid - Methyl Methacrylate Copolymer (1:1)), USP/NF (Methacrylic Acid Copolymer, Type A - NF) and JP (Methacrylic Acid Copolymer L) monographs. Glass transition temperature (Tg) is above 130°C (+/- 5°C).

Chemical/IUPAC name of LI 00 is"Poly(methacylic acid-co-methyl methacrylate) 1 : 1". It dissolves above pH 6.0 and targeted drug release area is jejunum.

EUDRAGIT^{®} L100-55 is described at the Ph.Eur (Methacrylic Acid - Ethyl Acrylate Copolymer (1:1) Type A), USP/NF (Methacrylic Acid Copolymer, Type C - NF) and JP (Dried

Methacrylic Acid Copolymer LD) monographs. Glass transition temperature (Tg) is 96°C (+/- 5°C).

Chemical/IUPAC name of L100-55 is "Poly(methacylic acid-co-ethyl acrylate) 1 : 1". It dissolves above pH 5.5 and targeted drug release area is duodenum.

EUDRAGIT^{®} S100 is described at the Ph.Eur (Methacrylic Acid - Methyl Methacrylate Copolymer (1:2)), USP/NF (Methacrylic Acid Copolymer, Type B - NF) and JP (Methacrylic Acid Copolymer S) monographs. Glass transition temperature (Tg) is above 130°C (+/- 5°C).

Chemical/IUPAC name of 5100 is"Poly(methacylic acid-co-methyl methacrylate) 1:2". It dissolves above pH 7.0 and targeted drug release area is ileum.

Preferably, pH dependent polymer soluble starting from pH 5.5 is selected from, but are not limited to, hypromellose phthalate, hypromellose acetate succinate, cellulose acetate phthalate, copolymer of methylmethacrylic acid and methyl methacrylate and the mixtures thereof as Eudragit derivatives; more preferably, Eudragit^{®} L100-55.

Preferably, pH dependent polymer soluble starting from pH 6.0 is selected from, but not limited to, copolymer of methylmethacrylic acid and methyl methacrylate, cellulose acetate phthalate, cellulose acetate succinate, hydroxypropyl methylcellulose phthalate, hydroxypropyl methylcellulose acetate succinate and the mixtures thereof as Eudragit derivatives; more preferably, Eudragit^{®} LI 00.

Preferably, pH dependent polymer soluble starting from pH 7.0 is selected from, but not limited to, copolymer of methylmethacrylic acid and methyl methacrylate, cellulose acetate phthalate, cellulose acetate succinate, hydroxypropyl methylcellulose phthalate, hydroxypropyl methylcellulose acetate succinate and the mixtures thereof as Eudragit derivatives; more preferably, Eudragit^{®} SI 00.

In a preferred embodiment, the present invention for the tablet formulation includes at least two pH dependent polymers located in the core matrix of the dosage form is at least 30% by weight of the composition. The third pH dependent polymer is in the coating layer and the range of this enteric coating is between 0.5% - 2.5% of the composition by weight of core tablet

The said invention is achieved by combining propiverine or one of its pharmaceutically acceptable salts with at least three pH dependent polymers, at least one hydrophilic polymer and at least one water soluble pore forming agent may further include one or more of other pharmaceutically acceptable excipients selected from, but not limited to, binder, lubricant, diluents, flavoring agent, sweetener, coloring agent, surfactant, stabilizing agent, viscosity agent, film coating agents or any combination thereof.

In a preferred embodiment, the controlled-release oral pharmaceutical composition comprising propiverine or pharmaceutically acceptable salt thereof characterized in that, said composition comprises at least two pH dependent polymers in the core matrix and an enteric coating in an amount of the range between 0.5% to 2.5% by weight of core tablet wherein pH dependent polymers in the core matrix are soluble starting from pH 6 Methacrylic Acid - Methyl Methacrylate Copolymer (1:1) and soluble starting from pH 5.5 Methacrylic Acid - Ethyl Acrylate Copolymer (1:1) Type A, wherein the amount of pH dependent polymers in matrix is at least 30% by weight of the composition, and the pH dependent polymer in enteric coating is Methacrylic Acid - Methyl 15 Methacrylate Copolymer (1:2).

The composition can comprise 4 mg to 60 mg of propiverine or a corresponding equivalent amount of propiverine salt or a mixture thereof.

The composition can also comprise propiverine hydrochloride as a propiverine salt.

The composition can also be used in the treatment of overactive bladder and urinary incontinence.

In one embodiment of the invention, the pharmaceutical composition comprises at least one lubricant, selected from the group consisting of calcium stearate, glycerine monostearate, magnesium stearate, stearic acid, glycerylpalmitostearate, glycerylbehenate, hydrogenated castor oil, hydrogenated vegetable oil, sodium lauryl sulphate, magnesium lauryl sulphate, sodium stearylfumarate, poloxamer, polyethylene glycol, sucrose ester of fatty acids, talc and any combination thereof.

In another preferred embodiment, the pharmaceutical composition comprises at least one binder, selected from the group consisting of carbomer, carboxymethylcellulose sodium, dextrin, dextrose, maltodextrin, gelatin, hydroxyethyl cellulose, hydroxyethylmethyl cellulose, hydroxypropyl cellulose, low substituted hydroxypropyl cellulose, ethylcellulose, methyl cellulose, hypromellose, magnesium aluminium silicate, methylcellulose, povidone, sodium alginate, starch, pregelatinised starch, liquid glucose, sucrose, tragacanth (gum benjamin), zein, acacia (gum arabic), alginic acid, guar gum, copovidone and any combination thereof.

In one embodiment of the invention, film coating agents include but not limited to hydroxypropylmethyl cellulose, ethyl cellulose, cellulose acetate, polyvinyl alcohol, acrylic and/or methacrylic co-polymers, resins, one or more plastifying agents (e.g., polyethylene glycol, propylene glycol, glycerin, triethyl citrate, diethyl phthalate, and mixtures thereof.).

In one embodiment of the invention, viscosity agents include but not limited to carboxymethyl cellulose sodium, carboxymethyl cellulose calcium, chitosan, colloidal silicon dioxide, gelatin, guar gum, xanthan gum, hydroxyethyl cellulose, hydroxypropyl cellulose, hydroxymethyl cellulose, hypromellose, maltodextrine, polyvinyl alcohol and the like or combinations thereof.

In one embodiment, the present invention is a pharmaceutical composition which is suitable for oral administration, such as tablet, film-coated tablet, dispersible tablet, orally disintegrating tablet, powder, granules or a combination thereof. Preferred dosage form is tablet. In a more preferred embodiment of the invention, the said pharmaceutical composition is formulated in a single dosage form for oral administration withan enteric coating. The enteric coated tablet of the present invention comprises a coating layer that is formed by spraying wherein the spraying solution contains a polymer dissolving at pH 7 or above, a lubricant such as a talc, and a film forming agent such as triethyl citrate, or the like. Water, ethanol, isopropyl alcohol and mixtures can be used as a solvent of the solution.

Another embodiment discloses a process of manufacturing controlled release formulation of propiverine or a pharmaceutically acceptable salt thereof. The tablet formulation may be produced with the techniques of direct compression, dry granulation and wet granulation. In the preferred embodiment of the present invention, direct compression method is applicable as well as wet granulation and dry granulation techniques. Water, ethanol, isopropyl alcohol and mixtures thereof can be used as solvents during wet granulation.

Direct compression technique has the least steps for tablet manufacturing and more advantageous for the industry therefore direct compression technique is usually the first approach while developing a new pharmaceutical formulation. Granulation techniques are applied if the powder properties of direct compression powder blend as powder flow, compressibility, homogeneity etc. are problematic. Said pharmaceutical invention is advantageous for the industry since formulation blends are very suitable for direct compression technique.

Said pharmaceutical composition is compressed into tablets after direct compression or dry granulation or wet granulation. The tablets may be film-coated in a conventional manner to achieve a controlled-release behave.

The controlled release formulation may also include one or more additional active ingredients.

Having described the invention with reference to certain embodiments, other embodiments will become apparent to the person skilled in the art from consideration of the specification. Certain specific aspects and embodiments of the invention will be further described in the following examples, which are provided solely for purposes of illustration and are not intended to limit the scope of the invention in any manner.

### Preparation of the composition

The composition may be produced in conventional manner by mixing the components. Below are examples of specific forms of the composition allowing various release profile of the active agent contained therein. All the excipients in the below compositions are mixed, sieved and compressed into tablets with the appropriate tablet weights.

### Examples

The dissolution conditions were designated considering the gastrointestinal pathway of orally applied solid dosage forms. The first pH condition in the dissolution method designated is similar to stomach as pH 1.2 and pH 5.8 and 6.8 conditions are chosen with respect to duodenum, jejunum and ileum regions of the GI tract.

The examples given below in the table are designed to get a controlled-release tablet having 0.5% - 2.5% enteric coating layer by weight of core tablet and one more out of this range, 3.0% by weight of core tablet. In all the examples, the quantitative and qualitative formulation of the core is the same. The unique and only difference of CR tablets is the amount of coating layer by weight of core tablet.

**Table 1: CR Tablet formulations with the same core tablet formulation and different amounts of coating layer**

| | **Formula-1** | **Formula-2** | **Formula-3** | **Formula-4** |
|---|---|---|---|---|
| **Ingredients** | w/w, % | w/w, % | w/w, % | w/w, % |
| Propiverine Hydrochloride | 7.5 | 7.5 | 7.5 | 7.5 |
| Other excipients | 92.5 | 92.5 | 92.5 | 92.5 |
| **Coating Layer** | **0.5** | **1.5** | **2.5** | **3.0** |

Having a composition of the coating layer shown in the following table was sprayed onto core tablets as above mentioned amounts

**Table 2: Composition of Coating Layer**

| **Ingredients** | **%** |
|---|---|
| Eudragit S100 | 4.3 |
| Triethyl citrate | 0.6 |
| Talc | 3.1 |
| Acetone | 35.1 |
| Isopropyl alcohol | 52.6 |
| Purified water | 4.4 |
| **Coating Layer** | **100%** |

The above mentioned tablet may be prepared by any method known. In present invention, core tablet is prepared by wet granulation and dry compaction, respectively. An enteric coating is applied to tablets after tablet compression. Coating layer as being a suspension is prepared in two step. In the first step, a solvent mixture comprising isopropylalcohol, acetone and deionized water is prepared. For the further steps this mixture is used as solvent. Eudragit S100 is added to the half of this mixture and mixed till to dissolve. This mixture is called excipient suspension. In a separate bowl, talc, triethyl citrate are mixed in the other half of the solvent mixture which is called spray suspension. Excipient suspension and spray suspension are mixed and filtered through 0.5 mm filter.

The prepared coating suspension is sprayed onto prepared core tablets as amounts mentioned in Table 1.

The composition of core tablet of all the examples are the same and the coating layer amounts are different from each other. The specified range to achieve suitable in vitro and in vivo results is 0.5% - 2.5% by weight of tablet. Moreover, there is also a tablet preparation with coating layer which is out of specified range.

**Table 3: In-vitro Dissolution results of Formulation I, II, III and IV**

| | **Dissolution results, %** | | | |
|---|---|---|---|---|
| **Time, h** | **Formulation I** | **Formulation II** | **Formulation III** | **Formulation IV** |
| **2** | 33 | 12 | 13 | 6 |
| **3** | 39 | 16 | 17 | 9 |
| **9** | 52 | 36 | 33 | 19 |
| **24** | 97 | 98 | 97 | 61 |

### Dissolution conditions;

2 hours in 0.1N HCI, 3^{rd} hour in pH 4.5 buffer, 9^{th} hour in pH 5.8 buffer and 24^{th} hour in pH 6.8 buffer, 100 rpm, Ph. Eur. Basket method at 100 rpm and 37°C±5°C.

In-vitro dissolution results of Formulation IV with an enteric coating amount as out of 0.5%-2.5% range are very low comparing with other formulations in specified range.

Final enteric coated tablets are used to conduct a bioequivalence study with a design described in the further.

An open-label, randomized, three treatment, six sequence, three period, single dose, cross over on a specified healthy subjects in fed conditions.

A single oral dose of test (T) or reference product (R) will be administered to study subjects in sitting posture at fixed time points with 240 ± 02 ml of water, at ambient temperature in each period as per randomization schedule. This activity will be followed by a mouth check to assess compliance to dosing.

After dosing healthy subjects with test and reference products, blood samples were taken according to pre-determined sampling schedule.

Subjects remained in upright position for 04 hours after dosing. During this period, subjects were seated and permitted to walk in an upright position for natural exigencies and study procedures such as blood sampling and vital parameter measurements.

For safety assessment, vital signs measurement and subject well-being assessment were done during subject check-in, housing and ambulatory.

Propiverine which is the analyte in plasma and was measured with a validated bio -analytical method. Primary pharmacokinetic parameters which are Cₘₐₓ and AUCo-ₜ were measured. According to Guideline on the Investigation on Bioequivalence published by EMA, to establish bioequivalence of the test product with that of reference product, 90% Confidence Interval (Cl) for the ratio (Test/Reference) of Least Square Means of the Ln transformed PK parameters (AUCo-ₜand Cₘₐₓ) must fall between 80.00% to 125.00%.

**Table 4: Bioequivalence study results**

| **Pharmacokinetic Parameters** | **90% Confidence Interval for Form. I vs Reference** |
|---|---|
| LN_Cₘₐₓ | 91.78% to 115.62% |
| LN_AUC₀₋ₜ | 93.49% to 113.32% |

| **Phamacokinetic Parameters** | **90% Confidence Interval for Form. III vs Reference** |
|---|---|
| LN_Cₘₐₓ | 107.38% to 123.48% |
| LN_AUC₀₋ₜ | 104.80% to 120.25% |

| **Phamacokinetic Parameters** | **90% Confidence Interval for Form. II vs Reference** |
|---|---|
| LN_Cₘₐₓ | 107.51% to 119.88% |
| LN_AUC₀₋ₜ | 107.00% to 119.95% |

| **Phamacokinetic Parameters** | **90% Confidence Interval for Form. IV vs Reference** |
|---|---|
| LN_Cₘₐₓ | 121.86% to 157.35% |
| LN_AUC₀₋ₜ | 102.21% to 119.51% |

In-vivo bioequivalence results of Formulation I, II, III that have enteric coating amount in 0.5%-2.5% range are in line with the guideline (80.00% to 125.00%).

In-vivo bioequivalence results of Formulation IV with an enteric coating amount as out of 0.5%-2.5% range are not in line with the guideline (80.00% to 125.00%).

## Claims

1. A controlled-release oral pharmaceutical composition comprising propiverine or pharmaceutically acceptable salt thereof **characterized in that**, said composition comprises at least two pH dependent polymers in the core matrix and an enteric coating in an amount of the range between 0.5% to 2.5% by weight of core tablet wherein pH dependent polymers in the core matrix are soluble starting from pH 6 Methacrylic Acid - Methyl Methacrylate Copolymer (1:1) and soluble starting from pH 5.5 Methacrylic Acid - Ethyl Acrylate Copolymer (1:1) Type A,
wherein the amount of pH dependent polymers in matrix is at least 30% by weight of the composition, and the pH dependent polymer in enteric coating is Methacrylic Acid - Methyl 15 Methacrylate Copolymer (1:2).

2. A controlled-release oral pharmaceutical composition according to any of the preceding claims, wherein it comprises an amount of 4 mg to 60 mg of propiverine or a corresponding equivalent amount of propiverine salt or a mixture thereof.

3. A controlled-release oral pharmaceutical composition according to claim 2 wherein the 20 composition comprises propiverine hydrochloride.

4. A controlled-release oral pharmaceutical composition according to any of the preceding claims for use in the treatment of overactive bladder and urinary incontinence.

## Patentansprüche

1. Eine orale pharmazeutische Zusammensetzung mit kontrollierter Freisetzung, bestehend aus Propiverin oder ein pharmazeutisch erträgliches/zulässiges Salz, **dadurch gekennzeichnet, dass** die Zusammensetzung mindestens zwei pH-abhängige Polymere in der Kernmatrix und einen magensaftresistente Beschichtung in einer Menge im Bereich von 0,5 bis 2,5 % nach Gewicht der Tablette enthält, wobei die pH-abhängigen Polymere in der Kernmatrix von ab pH 6 Methacrylsäure-Methylmethacrylat-Copolymer (1:1) löslich sind und von ab pH 5,5 Methacrylsäure-Ethylacrylat Copolymer (1:1) Typ A löslich sind, worin die Menge der pH-abhängigen Polymere in der Matrix mindestens 30 % nach Gewicht der Zusammensetzung beträgt und das pH-abhängige Polymer in der magensaftresistenten Beschichtung Methacrylsäure-Methylmethacrylat-Copolymer (1:2) ist.

2. Eine orale pharmazeutische Zusammensetzung mit kontrollierter Freisetzung nach einem der vorstehenden Ansprüche, wobei sie eine Menge von 4 mg bis 60 mg Propiverin oder eine entsprechende Menge an Propiverin-Salz oder eine Mischung davon enthält.

3. Eine orale pharmazeutische Zusammensetzung mit kontrollierter Freisetzung nach Anspruch 2, wobei die 20 Zusammensetzung Propiverin-Hydrochlorid enthält.

4. Eine orale pharmazeutische Zusammensetzung mit kontrollierter Freisetzung nach einem der vorstehenden Ansprüche zur Verwendung bei der Behandlung von überaktiver Blase und Harninkontinenz.

## Revendications

1. Composition pharmaceutique orale à libération contrôlée comprenant de la propivérine ou un sel pharmaceutiquement acceptable de celle-ci, **caractérisée par le fait que** ladite composition comprend au moins deux polymères dépendant du pH dans la matrice centrale et un enrobage entérique dans une quantité comprise entre 0,5 % et 2,5 % en poids du comprimé central où les polymères dépendants du pH dans la matrice centrale sont solubles à partir d'un pH de 6 Copolymère d'acide méthacrylique et de méthacrylate de méthyle (1:1) et solubles à partir d'un pH de 5,5 Copolymère d'acide méthacrylique et d'acrylate d'éthyle (1:1) de type A, où la quantité de polymères dépendants du pH dans la matrice est d'au moins 30 % en poids de la composition, et le polymère dépendant du pH dans l'enrobage entérique est le copolymère d'acide méthacrylique et de méthacrylate de méthyle (1:2).

2. Composition pharmaceutique orale à libération contrôlée selon toute réclamation précédente dans laquelle elle comprend une quantité de 4 mg à 60 mg de propivérine ou une quantité équivalente correspondante de sel de propivérine ou un mélange de ceux-ci.

3. Composition pharmaceutique orale à libération contrôlée selon la réclamation 2, dans laquelle la 20 composition comprend du chlorhydrate de propivérine.

4. Composition pharmaceutique orale à libération contrôlée selon toute réclamation précédente pour utilisation dans le traitement de la vessie hyperactive et de l'incontinence urinaire.
